# EUROPEAN PATENT APPLICATION

(11) **EP 2 071 019 A1**
(43) Date of publication of application: **17.06.2009**
(21) Application number: 07024355.5
(22) Date of filing: 15.12.2007
(51) Int. Cl.: C12N 1/14, C12P 7/64

(54) **Method for the cultivation of microoranisms of the order thraustochytriales**

(71) Applicant: Lonza AG, 3930 Visp (CH)
(72) Inventor: Luy, Markus, Dr., 3911 Ried-Brig (CH)
(74) Representative: Gleiss & Grosse

(57) **Abstract**

The present invention relates generally to the field of microorganism cell culture. It devises a cell culture medium for and a method of culturing microorganisms of the order Thraustochytriales, especially for the production of omega-3-fatty acids. Furthermore it devises the use of ammonium tartrate as nitrogen source for the cultivation of microorganisms of the order Thraustochytriales.

## Description

The present invention relates generally to the field of microorganism cell culture. It devises a cell culture medium for and a method of culturing microorganisms of the order Thraustochytriales, especially for the production of omega-3-fatty acids. Furthermore it devises the use of ammonium tartrate as nitrogen source for the cultivation of microorganisms of the order Thraustochytriales (Thraustochytriidea).

The present invention discloses methods to increase the total amount of PUFAs, especially omega-3-docosahexaenoic acid (DHA), in the biomass of microorganisms of the order Thraustochytriales by modifying the cell culture medium. The DHA amount in the biomass increases when specific salts, especially ammonium tartrate, are present in the cell culture medium. Since not only the total amount of DHA but also the total lipid amount is increased, the catching up of the product is simplified, resulting in better DHA yields.

There are different cell culture media for the cultivation of marine microorganisms of the order Thraustochytriales, especially of microorganisms belonging to the species Ulkenia. EP 0 935 667 describes the cultivation of Ulkenia cells in a medium containing potassium phosphate, ammonium sulfate, sodium sulfate and different chlorides. US2007/0141686 A1 describes a method for the cultivation of Thraustochytriales, wherein the cell culture medium is pH-stabilized using calcium carbonate. US2007/0054384 A1 describes a method for the cultivation of Thraustochytriales in a low salt medium without adding sodium salts and chloride salts. US 6,410,281 B1 describes a method for the cultivation of Thraustochytriales using a cell culture medium containing non-chloride-containing sodium salts, in particular sodium sulfate.

The microorganism belonging to the order Thraustochytriales (Thraustochytriidea) and especially to the genus Ulkenia is known to produce lipids containing omega-3-docosahexaenoic acid, also known as DHA, and/or omega-3-docosapentaenoic acid, also known as DPA.

Different polyunsaturated fatty acids (PUFAs) and especially omega-3 fatty acids are lipids known as being essential components of the human nutrition and as useful food additives. For example, PUFAs are known to lower the concentration of triglycerides in the serum and are therefore used as antilipaemics.

DHA and DPA are polyunsaturated fatty acids, which are used as food additives.

Present methods to produce DHA and/or DPA by cultivating Ulkenia result in unsatisfactory yields of lipids, especially of DHA and/or DPA or include complicated steps. There still exists a need for improved methods for the cultivation of microorganisms of the order Thraustochytriales.

Therefore, it was an object of the present invention to devise new methods for a simple and economically sensible cultivation of microorganisms belonging to the order Thraustochytriales for an increased production of PUFAs.

It is an object of the present invention to avoid the disadvantages of the prior art. It is a further object of the present invention to devise another method for enhancing the yield of PUFAs, especially DHA and/or DPA in Thraustochytriales cell culture. It is a further object of the present invention to devise another method for the production of high purity PUFAs, especially DHA and/or DPA.

These objects and further objects, which are not explicitly mentioned but are also disclosed to the person skilled in the art by the context of this description are solved according to the subject matter of the claims of the present invention.

The present invention solves the technical problem underlying the present invention by the provision of the methods, the cell culture medium, the use of ammonium tartrate and the products according to the claims.

The present invention solves the technical problem underlying the present invention especially by the provision of a method for the cultivation of a microorganism of the order Thraustochytriales, comprising the steps a) transferring the microorganism in a cell culture medium and b) culturing the microorganism in the cell culture medium, wherein the cell culture medium contains no ammonium sulfate.

In a preferred embodiment of the invention, the cell culture medium contains at least one ammonium salt, which is not ammonium sulfate. In a preferred embodiment of the invention, the cell culture medium contains ammonium tartrate. In a preferred embodiment of the invention, the cell culture medium contains ammonium tartrate but no ammonium sulfate.

The present invention solves the technical problem underlying the present invention furthermore by the provision of a method for the cultivation of a microorganism of the order Thraustochytriales, comprising the steps a) transferring the microorganism in a cell culture medium and b) culturing the microorganism in the cell culture medium, wherein the cell culture medium contains no ammonium sulfate but ammonium tartrate.

The present invention solves the technical problem underlying the present invention furthermore by the provision of a method for the cultivation of a microorganism of the order Thraustochytriales, comprising the steps a) transferring the microorganism in a cell culture medium and b) culturing the microorganism in the cell culture medium, wherein the cell culture medium contains ammonium tartrate.

In a particularly preferred embodiment of the above-identified method, it is foreseen subsequent to step b) to harvest, in particular to isolate polyunsaturated fatty acids, in particular DHA and/or DPA from the culture medium or the microorganism.

The present invention solves the technical problem underlying the present invention furthermore by the provision of a method for the production of at least one polyunsaturated fatty acid, comprising the steps a) transferring a microorganism of the order Thraustochytriales in a cell culture medium, b) culturing the microorganism in the cell culture medium and c) harvesting said polyunsaturated fatty acids from the microorganism and/or the cell culture, wherein the cell culture medium contains ammonium tartrate.

In one embodiment of the present invention, the polyunsaturated fatty acids are harvested from the microorganism and/or the cell culture in form of an oil comprising various lipids.

According to the present invention, it is understood herein that a fatty acid is an aliphatic monocarboxylic acid. Lipids are understood to be fats or oils including the glyceride esters of fatty acids along with associated phosphatides, sterols, alcohols, hydrocarbons, ketones, and related compounds.

According to the present invention, polyunsaturated fatty acids (PUFAs) are polyunsaturated long-chain fatty acids with a chain length greater C12 comprising at least two double bonds. PUFAs which can be produced according to the method of the present invention are preferably n-3 fatty acids and n-6 fatty acids, more preferably n-3 fatty acids.

In the sense of the present invention, n-3 fatty (omega-3 fatty acids) are understood to be polyunsaturated long-chain fatty acids with a chain length greater C12 comprising at least two ore more double bonds.

In a preferred embodiment of the present invention, the at least one polyunsaturated fatty acid (PUFA) is separated in a further step d) from other lipids.

In a preferred embodiment of the present invention, the at least one polyunsaturated fatty acid (PUFA) is a PUFA of high purity.

In a preferred embodiment of the present invention, the polyunsaturated fatty acids contain omega-3-docosahexaenoic acid (DHA) and/or omega-3-docosapentaenoic acid (DPA). In a preferred embodiment of the present invention, the polyunsaturated fatty acids contain omega-3-docosahexaenoic acid (DHA). In a preferred embodiment of the present invention, the polyunsaturated fatty acids contain omega-3-docosapentaenoic acid (DPA). In a preferred embodiment of the present invention, the polyunsaturated fatty acids contain omega-3-docosahexaenoic acid (DHA) and omega-3-docosapentaenoic acid (DPA). In a preferred embodiment of the present invention, the polyunsaturated fatty acids are omega-3-docosahexaenoic acid (DHA) and/or omega-3-docosapentaenoic acid (DPA). In a preferred embodiment of the present invention, the polyunsaturated fatty acids are omega-3-docosahexaenoic acid (DHA) and omega-3-docosapentaenoic acid (DPA). In a preferred embodiment of the present invention, the polyunsaturated fatty acid is omega-3-docosahexaenoic acid (DHA). In a preferred embodiment of the present invention, the polyunsaturated fatty acid is omega-3-docosapentaenoic acid (DPA).

The present invention provides the unexpected teaching to cultivate a marine organism of the order Thraustochytriales for the production of PUFAs using ammonium tartrate in the culture medium, which surprisingly results in significantly increased amounts of DHA. The use of other nitrogen sources, like ammonium chloride, results in an increase of the amount of e.g. lipid content, but on the other hand decreases the total productivity, which results in decreased amounts of the total biomass being not economically profitable. In view of this, it was unexpected to obtain high amounts of DHA and total lipids, while simultaneously the amount of total biomass lies in a conventional range. It was not to be expected that the methods according to the present invention to produce PUFAs in a microorganism of the order Thraustochytriales result in an increase of the total content of DHA.

In a preferred embodiment of the present invention, the microorganism of the order Thraustochytriales contains at least 28 weight-%, more preferably more than 28 weight-%, even more preferably more than 30 weight-%, most preferably more than 31 weight-% omega-3-docosahexaenoic acid (DHA) (based on the total dry weight of the microorganism). In a preferred embodiment of the present invention, the microorganism of the order Thraustochytriales contains in step c) at least 28 weight-% omega-3-docosahexaenoic acid (DHA) (based on the total dry weight of the microorganism). In a preferred embodiment of the present invention, the microorganism of the order Thraustochytriales contains in step c) more than 28 weight-% omega-3-docosahexaenoic acid (DHA) (based on the total dry weight of the microorganism). In a preferred embodiment of the present invention, the microorganism of the order Thraustochytriales contains in step c) more than 30 weight-% omega-3-docosahexaenoic acid (DHA) (based on the total dry weight of the microorganism). In a preferred embodiment of the present invention, the microorganism of the order Thraustochytriales contains in step c) more than 31 weight-% omega-3-docosahexaenoic acid (DHA) (based on the total dry weight of the microorganism).

In a preferred embodiment of the present invention, the microorganism of the order Thraustochytriales contains at least 60 weight-%, more preferably more than 60 weight-% lipids (based the total dry weight of the microorganism). In a preferred embodiment of the present invention, the microorganism of the order Thraustochytriales contains more than 65 weight-%, more preferably more than 70 weight-% lipids (based on the total dry weight of the microorganism). In a preferred embodiment of the present invention, the microorganism of the order Thraustochytriales contains in step c) at least 60 weight-% lipids (based on the total dry weight of the microorganism). In a preferred embodiment of the present invention, the microorganism of the order Thraustochytriales contains in step c) more than 60 weight-% lipids (based on the total dry weight of the microorganism). In a preferred embodiment of the present invention, the microorganism of the order Thraustochytriales contains in step c) more than 65 weight-% lipids (based on the total dry weight of the microorganism). In a preferred embodiment of the present invention, the microorganism of the order Thraustochytriales contains in step c) more than 70 weight-% lipids (based on the total dry weight of the microorganism).

In a preferred embodiment of the present invention, the microorganism of the order Thraustochytriales belongs to the genus Ulkenia. In a preferred embodiment of the present invention, the microorganism of the order Thraustochytriales belongs to the genus Thraustochytrium, in particular to the species Thraustochytrium aureum or the genus Schizochytrium or a mixture thereof. In another preferred embodiment the microorganism for use in the present methods is a dinoflagellate such as Crypthecodinium, in particular Crypthecodinium cohnii. In a preferred embodiment of the present invention, the microorganism of the order Thraustochytriales belongs to Ulkenia sp. SAM 2179 or Ulkenia sp. SAM 2180.

In a preferred embodiment of the present invention, the cell culture medium contains no ammonium sulfate. In a preferred embodiment of the present invention, the cell culture medium contains at least 0,6 g/litre and not more than 4,0 g/litre sulfate-ions.

In a preferred embodiment of the present invention, the cell culture medium contains no ammonium chloride. In a preferred embodiment of the present invention, the cell culture medium contains ammonium chloride.

In a preferred embodiment of the present invention, the cell culture medium contains a nitrogen source. In a preferred embodiment of the present invention, the cell culture medium contains a nitrogen source besides ammonium tartrate.

In a preferred embodiment of the present invention, the cell culture medium contains a nitrogen source selected from the group consisting of peptone, yeast extract, malt extract, meat extract, casamino acid, corn steep liquor, soybean cake, sodium glutamate, ammonium acetate, ammonium chloride, ammonium nitrate and mixtures thereof.

In a preferred embodiment of the present invention, the ammonium tartrate in the cell culture medium is used as nitrogen source. In a preferred embodiment of the present invention, ammonium tartrate is the only nitrogen source in the cell culture medium. In a preferred embodiment of the present invention, the cell culture medium contains as nitrogen source ammonium tartrate and one further salt selected from the group consisting of ammonium acetate, ammonium chloride, ammonium nitrate and mixtures thereof.

In a preferred embodiment of the present invention, the cell culture medium contains or does not contain natural or artificial sea water.

In a preferred embodiment of the present invention, the cell culture medium contains a carbon source. In a preferred embodiment of the present invention, the cell culture medium contains a carbon source selected from the group consisting of glucose, fructose, xylose, saccharose, maltose, soluble starch, fucose, glucosamine, dextran, oleic acid, fats, preferably such as soybean fat, oil, glutamic acid, molasses, glycerol, mannitol, sodium acetate and mixtures thereof.

In a preferred embodiment of the present invention, the cell culture medium contains, more preferably as micronutrients, substances selected from the group consisting of potassium phosphate, potassium dihydrogen phosphate, sodium sulfate, magnesium sulfate, iron sulfate, copper sulfate, magnesium chloride, calcium chloride, vitamins and mixtures thereof.

In a preferred embodiment of the present invention, the cell culture medium contains glucose, corn steep liquor, potassium dihydrogen phosphate, magnesium sulfate, calcium chloride, sodium chloride and ammonium tartrate.

In a preferred embodiment of the present invention, the carbon source may be added at a concentration of 15 to 250 g per litre of cell culture medium. Nitrogen sources, including ammonium tartrate, may be added at a concentration of 0,5 to 13 g per litre, more preferably to 7 g per litre cell culture medium. In a preferred embodiment, ammonium tartrate is present in the culture medium in an amount from 0,5 to 10 g per litre, preferably 0,5 to 7 g per litre, most preferably 2 to 7 g per litre of cell culture medium. Preferably, the amount of the nitrogen source is increased in correspondence with the increase in the amount of the carbon source. Preferably, the amount of the nitrogen source, especially the ammonium salt, most preferably the ammonium tartrate is adjusted to the amount of the carbon source.

In a preferred embodiment of the present invention, the cell culture medium contains precursors of DHA and/or DPA. In a preferred embodiment of the present invention, the cell culture medium contains substances selected form the group consisting of tetradecane, hexadecane, octadecane, oleic acid, linoleic acid, α-linoleic acid and mixtures thereof.

In a preferred embodiment of the present invention, the carbon sources, nitrogen sources, precursors or the like are added to the cell culture medium before or during the cultivation. The addition of these components may be carried out once, repeatedly or continuously.

In a preferred embodiment of the present invention, the cell culture medium has a pH between 3.0 and 8.0, more preferably between 4.0 and 7.0 before the cultivation of the microorganisms.

In a preferred embodiment of the present invention, the cell culture medium is sterilized, more preferably by autoclaving, before the microorganism is cultured in the cell culture medium.

In a preferred embodiment of the present invention, the microorganism is cultured in the cell culture medium for 1 to 12 days, more preferably 2 to 10 days. In a preferred embodiment of the present invention, the microorganism is cultured in the cell culture medium at a temperature of 10° C to 40°C, more preferably at a temperature of 18° C to 32°C, even more preferably at a temperature of 22° C to 30°C, most preferably a temperature of 27° C to 29°C. In a preferred embodiment of the present invention, the microorganism is cultured in the cell culture medium at a temperature of 28° C. In a preferred embodiment of the present invention, the microorganism is cultured with aeration-agitation. In a preferred embodiment of the present invention, the microorganism is cultured with shaking. In a preferred embodiment of the present invention, the microorganism is cultured in a stationary phase. In another preferred embodiment of the invention the microorganism is cultured in a stirred tank reactor.

The microorganism of the order Thraustochytriales are preferably cultured by inoculating a cell culture medium according to the present invention with a pre-culture of the microorganism.

Cultivation can preferably take place batchwise, in a fed-batch mode or continuously. Suitable cultivation methods are known to the person skilled in the art.

By the cultivation methods described in the present invention, lipids containing PUFA, especially DHA and/or DPA, are produced and accumulated in the cells of the microorganism of the order Thraustochytriales. When a liquid medium is used, the lipids containing the PUFA may be recovered from a culture, or a sterilized culture, during the cultivation period, from a culture, or a sterilized culture, at the end of cultivation, or from cultured cells, or dried cells, collected from any one of the above-mentioned cultures. As used herein, the term "culture" means cell cultures of the microorganism of the order Thraustochytriales and includes cultured cells, dried cultured cells and processed cultured cells as well as culture broth containing cells and culture supernatant.

DHA and/or DPA may be preferably isolated from lipids containing DHA and/or DPA recovered from cultured cells, as follows. Preferably, after cultivation, cells are collected from the culture by conventional solid/liquid separation means such as centrifugation and filtration. Preferably, the cells are extensively washed with water, and preferably, they are then dried. The drying of the cells is preferably carried out by freeze-drying, air-drying or the like. Preferably, the dried cells are then destroyed, for example, by means of dyno-mill or ultrasonication, and lipids are extracted from the cells preferably with an organic solvent, more preferably under nitrogen stream. Organic solvent such as ether, hexane, methanol, ethanol, chloroform, dichloromethane or petroleum ether are preferably used. Alternate extraction with methanol and petroleum ether or extraction with a mixed solvent system of chloroform/methanol/water is also preferably used. A high concentration of lipids containing DHA and/or DPA can be obtained by evaporating the organic solvent from the extract under a reduced pressure. In another preferred embodiment of the invention lipids containing DHA and/or DPA can be obtained by direct pressing of the dry or wet cell biomass.

Alternatively, the extraction is preferably carried out with wet cells. In this case, a solvent compatible with water such as methanol and ethanol, or a mixed solvent compatible with water consisting of the alcohol(s) and water and/or other solvents is preferably used. The other procedures are preferably the same as described above.

In a preferred embodiment of the present invention, the PUFAs are obtained in high yield. In a preferred embodiment of the present invention, the PUFAs are obtained in high purity. In a preferred embodiment of the present invention, the PUFAs are obtained from the microorganism. In a preferred embodiment of the present invention, the PUFAs are obtained from the cell culture medium following the cultivation. In a preferred embodiment of the present invention, the PUFAs are obtained from the microorganism and from the cell culture medium following the cultivation.

The produced PUFAs are preferably generally available and harvested in form of neutral fats, for example as triacylglycerides, or polar lipids such as, for example, phosphatidylcholine, phosphatidylethanolamine or phosphatidylinositol.

In a preferred embodiment of the present invention, the produced PUFAs, in particular DHA and/or DPA are harvested in form of a neutral lipid fraction. In a furthermore preferred embodiment, the PUFAs, in particular DHA or DPA, are harvested in form of a polar lipid fraction. In a furthermore preferred embodiment, the PUFAs, in particular DHA or DPA, are harvested in form of a total lipid fraction.

In a particularly preferred embodiment, the present invention also relates to oils harvested according to the present invention, in particular PUFA-containing oils or fractions thereof, such as polar lipid fractions, total lipid fractions or neutral lipid fractions containing the PUFAs according to the present invention, in particular DHA or DPA.

For the purpose of the present invention, the terms PUFA, n-3 fatty acid or n-3 active substances are understood to be all possible forms, in which the corresponding fatty acids can exist for example as free fatty acids as well as esters, triglycerides, phospholipids or other derivatives. All these substances are summarized in the present description and the terms are used synonymously. Furthermore, the PUFAs can be preferably converted and concentrated by means of chemical or biocatalytic transesterification, for example with the help of suitable enzymes, preferably lipases, before or after the isolation from the culture.

The isolation of the PUFAs from the fermented microorganisms or medium or oils and the analysis of the fatty acid spectrum is carried out using common procedures known to the person skilled in the art.

The present invention solves the technical problem underlying the present invention furthermore by the provision of a cell culture medium for the cultivation of a microorganism of the order Thraustochytriales, wherein the cell culture medium contains ammonium tartrate as nitrogen source.

In a preferred embodiment of the present invention, the cell culture medium is one described in the context of the methods described above.

The present invention solves the technical problem underlying the present invention furthermore by the provision of the use of ammonium tartrate as nitrogen source in a cell culture medium for the cultivation of a microorganism of the order Thraustochytriales.

The present invention solves the technical problem underlying the present invention furthermore by the provision of the use of ammonium tartrate as nitrogen source in a cell culture medium for the production of at least one polyunsaturated fatty acid by the cultivation of a microorganism of the order Thraustochytriales in the cell culture medium.

In a preferred embodiment of the present invention, the at least one polyunsaturated fatty acid is omega-3-docosahexaenoic acid (DHA) and/or omega-3-docosapentaenoic acid (DPA).

In a preferred embodiment of the present invention, the ammonium tartrate is used in a method described above. In a preferred embodiment of the present invention, the ammonium tartrate is used in a cell culture medium described above.

The present invention solves the technical problem underlying the present invention furthermore by the provision of oil having a content of at least 10 weight-% DHA, preferably at least 20 weight-% DHA and more preferably at least 30 weight-% DHA, produced using a method of the present invention. Preferably, the oil is subsequently isolated from the cell culture and or the cell culture medium available by the method of the present invention.

The present invention solves the technical problem underlying the present invention furthermore by the provision of DHA, produced by using a method of the present invention. Preferably, the DHA is subsequently isolated from the cell culture and or the cell culture medium available by the method of the present invention.

The present invention solves the technical problem underlying the present invention furthermore by the provision of DPA, produced by using a method of the present invention. Preferably, the DPA is subsequently isolated from the cell culture and or the cell culture medium available by the method of the present invention.

The present invention furthermore solves the technical problem underlying the present invention by the provision of biomass, whereby the biomass is produced by a method of the present invention.

Further preferred embodiments of the present invention are the subject matter of the subclaims.

The present invention is illustrated in more detail in the following examples. However, the cell culture medium and the method according to the present invention are not limited to the examples.

### Examples:

### Example 1: Cell culture medium containing ammonium tartrate (medium A):

| | |
|---|---|
| glucose (g/L) | 150,0 |
| corn steep liquor (g/litre) | 3,75 |
| KH₂PO₄ (g/litre) | 3,0 |
| NaCl (g/litre) | 0,8 |
| CaCl₂ x 2H₂O (g/litre) | 0,3 |
| MgSO₄ x 7H₂O (g/litre) | 1,5 |
| (NH₄)₂C₄H₄O₆ (g/litre) | 6,95 |

### Example 2: Cell culture medium containing ammonium chloride (medium B):

| | |
|---|---|
| glucose (g/litre) | 150,0 |
| corn steep liquor (g/litre) | 3,75 |
| KH₂PO₄ (g/litre) | 3,0 |
| NaCl (g/litre) | 0,8 |
| CaCl₂ x 2H₂O (g/litre) | 0,3 |
| MgSO₄ x 7H₂O (g/litre) | 1,5 |
| NH₄Cl (g/litre) | 4,03 |

### Example 3: Cultivation of microorganisms of the order Thraustochytriales

The cultivation of microorganisms of the order Thraustochytriales in two different cell culture media according to the present invention (media A and B) was compared with the cultivation of the same kind of microorganism in a cell culture medium of the state of the art (medium C). The ammonium salts ammonium tartrate and ammonium chloride were used in media A and B in an amount resulting in the same amount of ammonium ions as in the medium C.

As microorganism cells of Ulkenia sp. SAM 2179 were used.

For the production of PUFAs the microorganisms were cultivated as follows:

### 1. preparatory culture:

Following preparatory culture medium was used for all three cultures:

| | |
|---|---|
| glucose (g/litre) | 30,0 |
| yeast extract (g/litre) | 10,0 (Sensient) |
| Tropic Marin (g/litre) | 16,65 |
| (Dr. Bienert GmbH, Wartenberg) | |

pH 6,0 was adjusted with HCl.

The microorganisms were cultivated in 2 litre Erlenmeyer flasks without a baffle containing 350 ml of the preparatory culture medium for 48 h at 160 rpm and 25°C.

### 2. main culture:

After the incubation in the preparatory culture medium, the cultures were transferred into cell culture media A (according to the invention), B (according to the invention) or C (state of the art):
Cell culture medium A: see example 1.
Cell culture medium B: see example 2.
Cell culture medium C:

| | |
|---|---|
| glucose (g/L) | 150,0 |
| corn steep liquor (g/litre) | 3,75 |
| KH₂PO₄ (g/litre) | 3,0 |
| NaCl (g/litre) | 0,8 |
| CaCl₂ x 2H₂O (g/litre) | 0,3 |
| MgSO₄ x 7H₂O (g/litre) | 1,5 |
| (NH₄)₂SO₄ (g/litre) | 5,0 |

All three cell culture media contained 1,36 g/litre ammonium ions.

The pH > 4,0 was adjusted with NaOH (20 weight-% (w/v)) or KOH (20 weight-% (w/v)).

The microorganisms were cultivated in 10 litre cultures at 28°C and an aeration of 0,75 wm.

The microorganisms were cultivated until no glucose was present in the medium.

Table 1 shows the results of the different cultures.

**Table 1: results of the cultivation**

| | medium A | medium B | medium C* |
|---|---|---|---|
| ammonium in the medium (g/litre) | 1,36 | 1,36 | 1,36 |
| DHA mass ( weight-%) | 31,8 | 32,9 | 27,3** |
| DHA area (weight-%) | 47,9 | 48,2 | 47,7 |
| DHA (g/litre) | 20,0 | 14,6 | 18,0 |
| DBM (g/litre) | 63,0 | 44,2 | 65,9 |
| oil/DBM ( weight-%) | 69,9 | 71,9 | 60,2 |

| | | | |
|---|---|---|---|
| DBM: dry biomass *: mean from 12 independent cultures **: lowest value 26,0 weight-%; highest value 28,7 weight-% | | | |

Different ammonium sources were used for the cell culture media A and B, whereby the absolute ammonium amount in the media was equal in media A, B and C.

The use of ammonium tartrate (medium A), but also the use of ammonium chloride (medium B), instead of ammonium sulfate (medium C) resulted in a drastic increase of the DHA mass- weight-% of at least 15 weight-% (proportion of DHA in the dry biomass), while the quality of the fatty acid spectra was not influenced (similar DHA area weight-% values).

The total amount of DHA was increased using ammonium tartrate in the cell culture medium. This was especially the result of the similar amount of the total dry biomass compared to the cultivation with medium C, since this leads to an increased total DHA amount, when the DHA content is increased.

Also the content of the total oil in the dry biomass was increased using ammonium tartrate instead of ammonium sulfate.

## Claims

1. A method for the cultivation of a microorganism of the order Thraustochytriales comprising the steps
a) transferring the microorganism in a cell culture medium and
b) culturing the microorganism in the cell culture medium,
wherein the cell culture medium contains ammonium tartrate.

2. The method for the production of at least one polyunsaturated fatty acid, comprising the steps
a) transferring a microorganism of the order Thraustochytriales in a cell culture medium,
b) culturing the microorganism in the cell culture medium,
c) harvesting said polyunsaturated fatty acids from the microorganism and/or the cell culture,
wherein the cell culture medium contains ammonium tartrate.

3. The method according to claim 2, wherein the at least one polyunsaturated fatty acid is separated in a further step d) from other lipids.

4. The method according to claims 2 or 3, wherein the polyunsaturated fatty acids contain omega-3-docosahexaenoic acid (DHA) and/or omega-3-docosapentaenoic acid (DPA).

5. The method according to claims 2 or 3, wherein the polyunsaturated fatty acids are omega-3-docosahexaenoic acid (DHA) and/or omega-3-docosapentaenoic acid (DPA).

6. The method according to claims 2 or 3, wherein the polyunsaturated fatty aid is omega-3-docosahexaenoic acid (DHA).

7. The method according to any of the preceding claims, wherein the microorganism of the order Thraustochytriales contains in step c) at least 28 weight-% omega-3-docosahexaenoic acid (DHA) based on the total dry weight of the microorganism.

8. The method according to any of the preceding claims, wherein the microorganism of the order Thraustochytriales contains in step c) at least 60 weight-% lipids based on the total dry weight of the microorganism.

9. The method according to any of the preceding claims, wherein the microorganism of the order Traustochytriales belongs to the genus Ulkenia.

10. The method according to any of the preceding claims, wherein the ammonium tartrate in the cell culture medium is used as nitrogen source.

11. The method according to any of the preceding claims, wherein ammonium tartrate is the only nitrogen source in the cell culture medium.

12. The method according to any of the preceding claims, wherein the cell culture medium contains from 0,6 g/litre to 4,0 g/litre sulphate-ions.

13. Cell culture medium for the cultivation of a microorganism of the order Thraustochytriales, wherein the cell culture medium contains ammonium tartrate as nitrogen source.

14. The use of ammonium tartrate as nitrogen source in a cell culture medium for the cultivation of a microorganism of the order Thraustochytriales.

15. The use of ammonium tartrate as nitrogen source in a cell culture medium for the production of at least one polyunsaturated fatty acid by the cultivation of a microorganism of the order Thraustochytriales in the cell culture medium.

16. The use according to claim 15, wherein the at least one polyunsaturated fatty acid is omega-3-docosahexaenoic acid (DHA) and/or omega-3-docosapentaenoic acid (DPA).

17. The use according to any one of claims 14 to 16, wherein the microorganism of the order Thraustochytriales belongs to the genus Ulkenia.
